# EUROPÄISCHE PATENTSCHRIFT

(11) **EP 0 826 362 B1**
(45) Veröffentlichungstag und Bekanntmachung des Hinweises auf die Patenterteilung: **09.01.2002**
(21) Anmeldenummer: 97114426.6
(22) Anmeldetag: 21.08.1997
(51) Int. Cl.: A61K 7/42

(54) **Kosmetisches Mittel mit polymergebundenen Benzophenonchromophoren**
Cosmetics with benzophenone chromophores bound on polymers
Cosmétique avec des benzophenones chromophores liées à des polymères

(30) Priorität: 26.08.1996 DE 19634399
(43) Veröffentlichungstag der Anmeldung: 04.03.1998
(73) Patentinhaber: BASF AKTIENGESELLSCHAFT, 67056 Ludwigshafen (DE)
(72) Erfinder: Keller, Harald, Dr., 67069 Ludwigshafen (DE); Sperling-Vietmeier, Karin, Dr., 67433 Neustadt (DE); Westenfelder, Horst, 67435 Neustadt (DE)

(56) Entgegenhaltungen:
- WO-A-96/14826
- US-A- 3 341 493
- US-A- 3 956 244

## Beschreibung

Die vorliegende Erfindung betrifft kosmetische Mittel mit polymergebundenen Benzophenonchromophoren zum Schutz der Haut und der Haare vor UV-Strahlung.

Bei kosmetischen Sonnenschutzmitteln, die auf der Haut aufgetragen werden, ist häufig eine gute Wasserfestigkeit wünschenswert. Diese Eigenschaft wird insbesondere gewünscht, wenn häufiger Kontakt der Haut mit Wasser oder wäßrigen Flüssigkeiten wie Schweiß stattfindet. Um die gewünschte Wasserfestigkeit zu erreichen, werden solchen Mitteln häufig Polymere zugesetzt.

Eine weitere Möglichkeit besteht darin, die UV-absorbierenden Gruppen kovalent mit einem Polymer zu verknüpfen.

In WO 89/4824 werden Copolymere aus dem UV-absorbierenden Styrol und Maleinsäureanhydrid, Vinylpyrrolidon oder Acrylaten beschrieben. Diese Copolymere besitzen eine hohe Wasserlöslichkeit und eine geringe Hautpenetration.

In JP 60/88066 werden UV-absorbierende Benzophenonderivate beschrieben, die über einen Urethanspacer an (Meth)acrylate gebunden sind. Diese Polymere eignen sich als Material für Kontaktlinsen und Brillengläser.

Neben der guten Wasserfestigkeit sollen die kosmetischen Mittel jedoch eine Reihe von anderen Eigenschaften wie geringe Hautpenetration, geringer Gehalt an Restmonomeren, die aus toxikologischen und olfaktorischen Gründen zu vermeiden sind, gute Verarbeitbarkeit, Mischbarkeit und Stabilität mit weiteren Komponenten kosmetischer Mittel, erfüllen.

Gefunden wurden kosmetische Mittel, enthaltend ein Polymer mit der sich wiederholenden Struktureinheit (I) 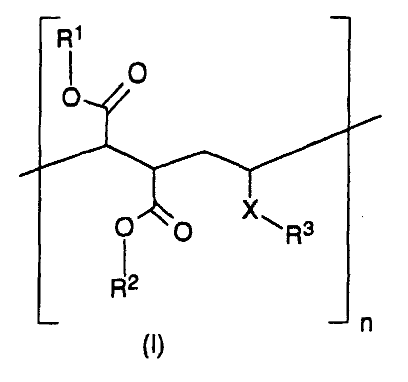 wobei
- R¹=: 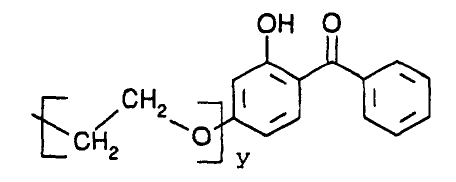 mit y = 0 - 6,
- R²=: R¹, H, Alkalimetall, Ammonium
- R³=: C3-C30-Alkyl,-CO-R4 mit
- R⁴=: C3-C30-Alkyl
- X=: O oder CH₂
- n =: 2-400
bedeuten.

Die Polymere für die erfindungsgemäßen kosmetischen Mittel lassen sich herstellen, indem man in einer ersten Stufe a) ein Copolymer aus Maleinsäureanhydrid und einem Olefin der Formel (II) 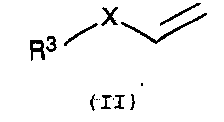 herstellt. Der Rest R³ hat dabei die Bedeutung C3-C30 Alkyl, bevorzugt C16-C22 Alkyl, X kann O oder CH₂ bedeuten. Besonders bevorzugte Polymere sind solche mit X=CH₂ und R³=C₁₉H₃₉.

Die Polymerisation erfolgt vorteilhaft, indem man das Olefin (II) vorlegt und unter Schutzgas bei 80° bis 150°C· das Maleinsäureanhydrid und einen Starter getrennt zudosiert. Als Starter können die üblichen Azostarter wie Azoisobutyronitril oder Peroxide wie Dibenzoylperoxid oder tertiär-Butyl-2-ethyl-perhexanoat in Mengen von 1 bis 15 % verwendet werden.

Anschließend setzt man das in a) erhaltene Copolymer mit dem Benzophenonderivat (III) 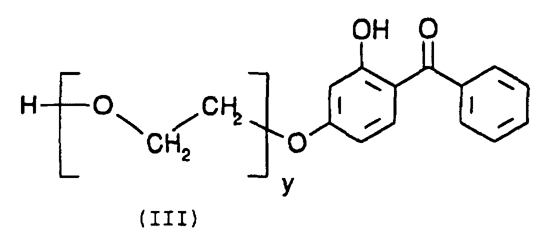 wobei y = 0 - 6 bedeutet, um. Dabei wird die Anhydridbindung gespalten und das Benzophenonderivat über eine Esterbindung an das Polymerrückgrat gebunden.

Neben den Benzophenon-Monomeren können weitere vinylische Comonomere wie z. B. Vinylpyrrolidon, Vinylacetat, 1-Olefine, Acrylate und Methacrylate einpolymerisiert werden.

Die Benzophenonderivate (III) sind bekannt oder lassen sich aus bekannten Ausgangsverbindungen mit üblichen Methoden darstellen.

Das nach Stufe b) erhaltene Polymer wird dann mit in der Kosmetik üblichen Hilfs- und Zusatzstoffen je nach Anwendungszweck formuliert. Beispiele geeigneter Hilfs-und Zusatzstoffe für Sonnenschutzmittel sind bei W. Umbach, Kosmetik, Georg-Thieme-Verlag Stuttgart, 1988 beschrieben.

Die erfindungsgemäßen kosmetischen Mittel zeichnen sich durch ihre hohe Wasserbeständigkeit, gute Hautverträglichkeit und geringe Hautpenetration aus.

Die Erfindung wird durch die folgenden Beispiele weiter veranschaulicht.

### Beispiel 1

### Herstellung eines UV-Licht absorbierenden Polymeren

200 g eines alternierenden Copolymeren aus Maleinsäureanhydrid und 1-Olefin mit einer Kettenlänge von C₂₀ bis C₂₄ , 203 g 1-(2-Hydroxy-4-(2-hydroxyethyl)oxy)benzophenon und 4 g Polyphosphorsäure werden 8h bei 140° C gerührt. Die Reaktionsmischung wird auf 60° C abgekühlt und mit 350 ml Tetrahydrofuran verdünnt. Anschließend wird die Polymerlösung in 2.5 1 Ethanol gefällt.
Ausbeute: 244 g
Erweichungspunkt: 49° C
Molekulargewicht nach GPC:Mn = 5790, Polydispersität = 2.6
UV:
   332 nm E¹₁= 138
   294 nm E¹₁= 219

### Beispiel 2

### Herstellung eines Sonnenschutzmittels

- Phase A:: 16 g des in Beispiel 1 hergestellten Polymers, 5 g Finsolv® TN, 10 g Witconol® APM und 0.5 g Nip Nip® werden bei 60° C gemischt und nach Abkühlen mit 1 g Cremophor RH40® gemischt.
- Phase C:: 0.3 g Pemulen® TR1, 5 g 1,2-Propylenglykol und 65 g Wasser werden gemischt. Danach werden 0.2 g Tylose® H4000 eingerührt.

Die Phase A wird in die Phase C eingerührt und durch Zugabe von 0.3 g Triethanolamin neutralisiert.

## Patentansprüche

1. Kosmetisches Mittel, enthaltend ein Polymer mit der sich wiederholenden Struktureinheit (I) 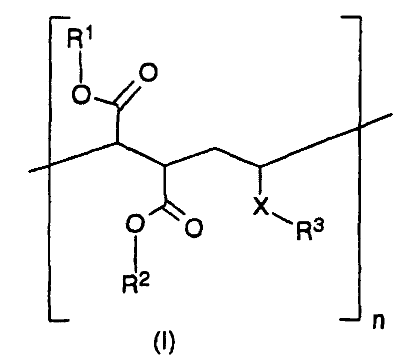 wobei
R¹ = 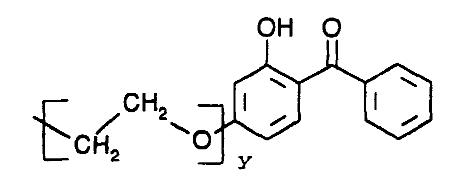 mit y = 0 - 6,
R² = R¹, H, Alkalimetall, Ammonium,
R³= C3-C30-Alkyl,-CO-R4 mit
R⁴= C3-C30-Alkyl,
X= O oder CH₂,
n= 2-400
bedeuten.

2. Kosmetisches Mittel nach Anspruch 1, **dadurch gekennzeichnet, daß** R³ für C₁₉H₃₉ und X für CH₂ steht.

3. Verfahren zur Herstellung eines kosmetischen Mittels nach Anspruch 1, indem man
a) ein Copolymer aus Maleinsäureanhydrid und einem Olefin der Formel (II) 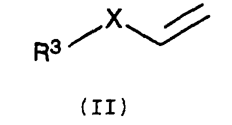 wobei die Reste, die in Anspruch 1 angegebene Bedeutung besitzen, herstellt,
b) dieses Copolymer mit dem Benzophenonderivat (III) 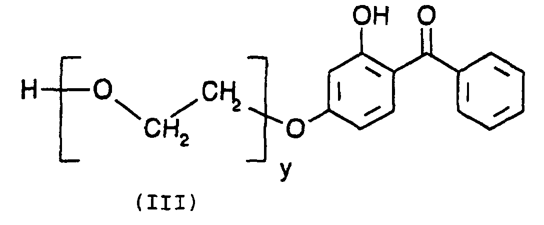 wobei y = 0 bis 6 bedeutet, umsetzt, und
c) das nach b) erhaltene Copolymer mit üblichen Hilfs- und Zusatzstoffen formuliert.

## Claims

1. A cosmetic composition comprising a polymer with the repeating structural unit (I) 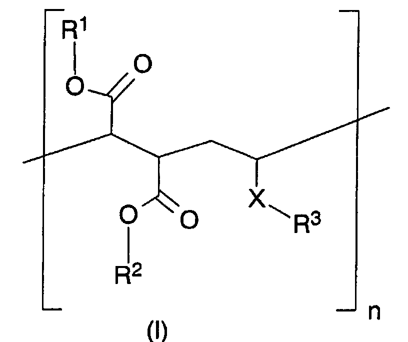 where
R¹ is 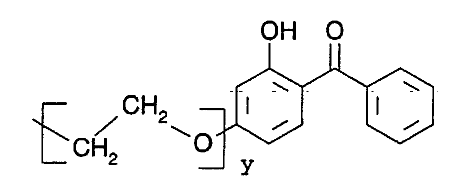 where y is 0 - 6,
R² is R¹, H, alkali metal, ammonium,
R³ is C3-C30-alkvl,-CO-R4 with
R⁴ = C3-C30-alkyl,
X is O or CH₂,
n is 2-400.

2. Cosmetic composition as claimed in claim 1, wherein R³ is C₁₉H₃₉ and X is CH₂.

3. Process for preparing a cosmetic composition as claimed in claim 1, by
a) preparing a copolymer from maleic anhydride and an olefin of the formula (II) 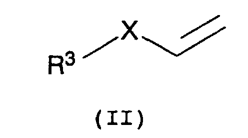 where the radicals have the meanings stated in claim 1,
b) reacting this copolymer with the benzophenone derivative (III) 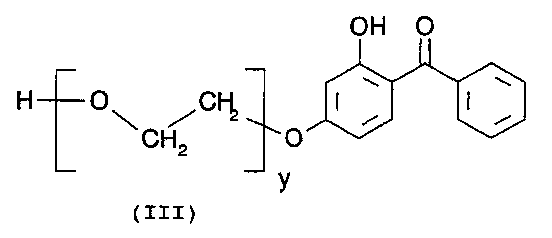 where y is 0 to 6, and
c) formulating the copolymer obtained in b) with conventional ancillary substances and additives.

## Revendications

1. Produit cosmétique contenant un polymère à motif de structure répété I 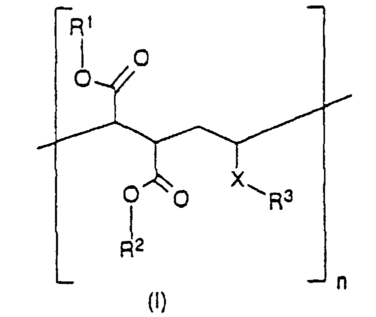
dans lequel R¹ = 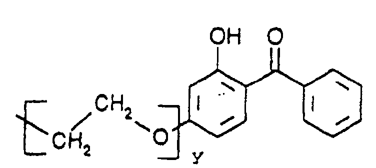 où y = 0 à 6,
R² = R¹, H, métal alcalin, ammonium,
R³ = alkyle en C3-C30, -CO-R⁴ avec
R⁴ = alkyle en C3-C30,
X = O ou CH₂,
n = 2 à 400.

2. Produit cosmétique selon la revendication 1 **caractérisé par le fait que** R³ représente C₁₉H₃₉ et X représente CH₂.

3. Procédé pour la préparation d'un produit cosmétique selon la revendication 1, selon lequel
a) on prépare un copolymère de l'anhydride maléique et d'une oléfine de formule II 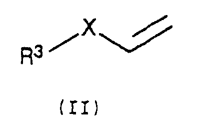 dans laquelle les symboles ont les significations indiquées dans la revendication 1,
b) on fait réagir ce copolymère avec le dérivé de benzophénone III 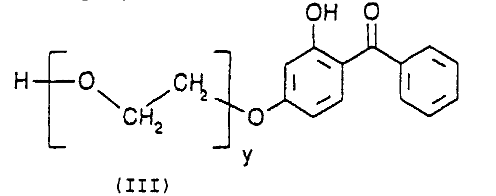 dans lequel y = 0 à 6, et
c) on forme un produit cosmétique avec le copolymère obtenu en b) ci-dessus et des produits auxiliaires et additifs usuels.
